# EUROPEAN PATENT APPLICATION

(11) **EP 4 014 993 A1**
(43) Date of publication of application: **22.06.2022**
(21) Application number: 20215987.7
(22) Date of filing: 21.12.2020
(51) Int. Cl.: A61K 39/395, A61P 21/02

(54) **RITUXIMAB FOR TREATMENT OF POLYMYALGIA RHEUMATICA**

(71) Applicant: Stichting Sint Maartenskliniek, 6574 NA Ubbergen (NL)
(72) Inventor: Marsman, Diane Emile, 6574 NA Ubbergen (NL); den Broeder, Nathan, 6574 NA Ubbergen (NL); van den Hoogen, Franciscus Hendrikus Josephus, 6574 NA Ubbergen (NL); den Broeder, Alfonso Abigael, 6574 NA Ubbergen (NL); van der Maas, Aatke, 6574 NA Ubbergen (NL)
(74) Representative: De Vries & Metman

(57) **Abstract**

The disclosure relates to a novel treatment of polymyalgia rheumatica (PMR), wherein the patient is administered rituximab (RTX). In a preferred embodiment, the patient is also administered a glucocorticoid (GC) such as prednisone or prednisolone. In the this case, the rituximab can act as a glucocorticoid sparing agent.

## Description

### FIELD OF THE INVENTION

The present invention pertains to the treatment of polymyalgia rheumatica

### BACKGROUND

Polymyalgia rheumatica (PMR) is a debilitating inflammatory rheumatic inflammatory disorder that causes muscle pain and stiffness, primarily in the neck, bilateral shoulder, upper arms, and hip girdle. Diagnosis is mostly based upon assessment of clinical symptoms, including pain and stiffness in the shoulder and pelvic girdle, muscle tenderness of the arms and legs, sometimes occurring in combination with fever, weight loss, and fatigue. It is most commonly found in elderly patients of over 50 years of age where it affects 0.1 to 0.5 % of the population. The cause of PMR is not well understood, although various reports suggest an influence of genetic, viral infections and/or autoantigens as possible triggers. Left untreated, PMR can lead to morbidity and/or a significant reduction in quality of life (QOL).

Glucocorticoids (GC), such as prednisone, methylprednisone prednisolone, methylprednisolone, fludrocortison, dexamethason, hydrocortison or deflazacort, are currently the cornerstone of polymyalgia rheumatica treatment. Routes of administration of GC can be oral, intravenous, intramuscular, intra-articular or topical. However, 29-45% of patients do not sufficiently respond to GC treatment after 3-4 weeks and only 33-50% of patients treated in secondary clinics achieve sustained GC-free remission after 2 years. There is thus a need for an alternative therapy that improves response rate and leads to faster GC-free remission in a larger portion of the patient population.

GC related side effects occur in about 50% of patients and can be a significant burden for patients, especially in prolonged treatment with GC. These side effects include osteoporosis, infections, hypertension, high cholesterol, weight gain, diabetes mellitus, peptic ulcers, cataracts as well as depression or other disturbances in emotional well-being. These side effects may be denoted in the art as GC-related adverse events. As a result, there is a need to develop alternative therapies that reduce the occurrence of side effects or, preferably, that do not have side effects at all.

In addition, there are patients that suffer besides PMR from other illnesses. Said patients may have a contra-indication for GC due to their comorbidities. This group of patients is becoming more prominent as the risk of comorbidities increases with age. These patients are in need of an alternative treatment that at least relies less on GCs and preferably does not use GCs at all.

Unfortunately, there is a lack of alternative treatments for PMR. There is hence an unmet need for an alternative treatment that at least reduces the dependency on GCs and preferably removes the dependency of GC.

Furthermore, a large subset of patients - around 50 % - experiences one or more flares during GC tapering (i.e. the gradual lowering of the GC dose with the aim to gradually stop administering GCs). These patients experience a sudden reoccurrence of (the severity of) the symptoms and therefore need an increase in GC dose. This often leads to a substantially prolonged treatment duration and as a result about 40% of patients need GC longer than four years. Hence, there is a need to reduce the occurrence and severity of flares that occur during GC tapering.

### SUMMARY

The inventors have found that rituximab can be used to treat patients suffering from polymyalgia rheumatica. This surprising discovery fulfils a long-felt need for an alternative treatment that reduces the dependency on glucocorticoids that the current PMR treatment has. The rituximab can be administered alone or - if desired - in combination with another medicament such as a glucocorticoid. In case that the rituximab is administered as sole medicament - or at least without a glucocorticoid - the patient receiving the rituximab will experience less side effects and/or less severe side effects. Also it helps to suppress flares such as often found during GC treatment, to reduce morning stiffness and pain and to reduce the duration of the treatment.

Also in case that the rituximab is administered along with a glucocorticoid there are multiple benefits. The treatment will be more effective and can therefore be shortened. It helps to lower the GC dose meaning that the occurrence and severity of side effects due to the GC treatment will decrease. It further helps to reduce the occurrence of flares to the point of not experiencing any single flare during a treatment. Also it helps to achieve GC-free remission more quickly.

### LIST OF PREFERRED EMBODIMENTS

1. Rituximab for use in the treatment of polymyalgia rheumatica.
2. Rituximab for use according to embodiment 1, wherein the treatment further comprises the administration of a glucocorticoid, preferably wherein the rituximab is used to lower the glucocorticoid dose and/or as a glucocorticoid sparing agent.
3. Rituximab for use according to embodiment 2, wherein the rituximab is used to reduce the duration of the treatment with the glucocorticoid.
4. Rituximab for use according to embodiment 2 or 3, wherein the glucocorticoid sparing comprises a 10 - 52 week, preferably 12 - 42 week, more preferably 14 - 32 week, even more preferably 15 - 25 week glucocorticoid tapering scheme.
5. Rituximab for use according to any of the previous embodiments, wherein the patient diagnosed with polymyalgia rheumatica has a contra-indication for treatment with glucocorticoids, a strong patient preference against treatment with glucocorticoids and/or a bad prognosis with regard to glucocorticoid tapering.
6. Rituximab for use according to any of the previous embodiments, wherein the rituximab is used to reduce the incidence of flares.
7. Rituximab for use according to any of the previous embodiments, wherein the rituximab is used to reduce the disease duration of polymyalgia rheumatica.
8. Rituximab for use according to any of the previous embodiments, wherein the rituximab is used to reduce the recurrence of polymyalgia rheumatica.
9. Rituximab for use according to any of the previous embodiments, wherein the rituximab is used to lower the polymyalgia rheumatica activity score or the polymyalgia rheumatica disease activity score.
10. Rituximab for use according to any of the previous embodiments, wherein the rituximab is administered in less than 5 doses.
11. Rituximab for use according to any of the previous embodiments, wherein the rituximab is administered as a single dose, preferably within 4 weeks after diagnosis of polymyalgia rheumatica.
12. Rituximab for use according to any of the previous embodiments, wherein the total dose of rituximab is 200 - 5000 mg
13. Rituximab for use according to any of the previous embodiments, wherein the dose of rituximab is 50 - 3000 mg per course.
14. Rituximab for use according to any of the previous embodiments, wherein the rituximab is administered intravenously or by subcutaneous injection.
15. Rituximab for use according to any of embodiments 2 - 14, wherein the glucocorticoid is administered orally.
16. Rituximab for use according to any of embodiments 2 - 15, wherein the glucocorticoid is cortisone, dexamethasone, hydrocortisone, methylprednisolone, prednisone, methylprednisone, prednisolone or methylprednisolone, triamcinolone, fludrocortisone, dexamethasone, or hydrocortisone, preferably wherein the glucocorticoid is prednisone, methylprednisone, prednisolone or methylprednisolone.
17. Rituximab for use according to any of the previous embodiments, wherein the treatment further comprises the administration of methotrexate, preferably in a dose of 5 - 50 mg/week, and/or leflunomide, preferably in a dose of 30 - 300 mg/week.
18. Rituximab for use according to any of the previous embodiments, wherein the patient receives premedication, preferably wherein the premedication comprises methylprednisolone, acetaminophen and/or antihistamines.

### DETAILED DESCRIPTION

The present invention concerns rituximab for use in the treatment of polymyalgia rheumatica.

Depending on the jurisdiction, the present invention may also be worded as the use of rituximab in the manufacture of a medicament for the treatment of polymyalgia rheumatica.

Alternatively, depending on the jurisdiction, the present invention may be worded as a method for treating a patient with rituximab, wherein the patient is suffering from polymyalgia rheumatica.

Rituximab (RTX) is a chimeric monoclonal antibody (mAb) targeting the CD20 antigen. It acts by depleting B-cells and was originally developed for the treatment of B-cell non-Hodgkin's lymphoma (NHL). Rituximab is nowadays used to treat a number of diseases including NHL, chronic lymphocytic leukemia, and rheumatoid arthritis. Also it is used for various treatments relating to cancer. As a result, there is sufficient clinical experience with RTX and it is considered to have a relatively safe adverse events profile. Furthermore, it is known that RTX is well tolerated by patients with other inflammatory rheumatic diseases such as rheumatoid arthritis.

The treatment with rituximab has multiple benefits which includes a reduction in side-effects of the treatment, such as GC-related adverse events, a reduction in duration of the disease, a reduction in disease severity and/or a reduction in disease symptom severity. Hence rituximab can be used for the treatment of PMR wherein the side-effects of the PMR treatment are reduced. It is hereby understood that reduced side effects implies that the side effects are reduced compared to the current standard treatment, which is a GC treatment wherein rituximab is not administered. Rituximab can be used to reduce the duration of treatment of polymyalgia rheumatica. It is hereby understood that reduce the duration of treatment of polymyalgia rheumatica implies that the duration of the treatment is reduced compared to the current standard treatment, which is a GC treatment wherein rituximab is not administered. Also a treatment with rituximab can be used to reduce the severity of polymyalgia rheumatica and/or reduce the symptom severity of polymyalgia rheumatica. Here it is understood that reducing the severity of polymyalgia rheumatica and/or reducing the symptom severity of polymyalgia rheumatica implies that the severity of polymyalgia rheumatica and/or the symptom severity of polymyalgia rheumatica is reduced compared to the current standard treatment, which is a GC treatment wherein rituximab is not administered. It is furthermore understood that the treatment with rituximab is especially suitable for patients in need thereof, such as polymyalgia rheumatica patients in need of a treatment for polymyalgia rheumatica.

The treatment with rituximab is suitable for newly and recently diagnosed PMR patients as well as for relapsing PMR patients, whereby newly and recently diagnosed PMR patients are particularly preferred. It is hereby understood that a PMR diagnosis is preferably established according to the 2012 EULAR/ ACR PMR classification. In this light, a newly and recently diagnosed PMR patient may be a patient who has received a polymyalgia rheumatica diagnosis fulfilling the 2012 EULAR/ ACR classification criteria in the last 3 months. The term a relapsing patient is used in its normal meaning. It may hence be a patient that is being treated such that the PMR symptoms are decreasing and where the patient experiences a sudden increase in symptoms. In other words, a relapsing patient may be patient that has been diagnosed with PMR, that is receiving a treatment whereby the dose of the medication (such as GC) is gradually lowered and where an increase in symptoms requires an increase in the dose of the medication (such as GC).

In this light a relapsing PMR patient may be characterized by the following criteria:
- polymyalgia rheumatica fulfilling the 2012 EULAR/ ACR classification criteria, and
- clinical relapse from a GC dose < 7.5 mg/day to GC dose of ≥ 7.5mg/day.

The treatment with rituximab may be considered especially suitable for elderly patients as these patients are most often diagnosed with PMR. It is herein understood that elderly patients are those patients who are at least 50 years of age, more preferably at least 60 years of age and most preferably at least 65 years of age.

Alternatively, the treatment with rituximab may be considered especially suitable for patients having a body-mass index of at least 25, more preferably at least 27, more preferably at least 29 and most preferably at least 30. Hereby it is understood that the body-mass index can be calculated by dividing the weight in kg by the square in height in meters (i.e. bodymass (kg) / height (m)²).

In a preferred embodiment, the treatment of polymyalgia rheumatica includes the administration of a glucocorticoid (GC). Glucocorticoids (GCs), which are also denoted as glucocorticosteroids, are a class of corticosteroids that bind to the glucocorticoid receptor. Glucocorticoids are part of the feedback mechanism in the immune system, which reduces certain aspects of immune function, such as inflammation.

Hence, in this embodiment, the invention can be denoted as rituximab and a glucocorticoid for use in the treatment of polymyalgia rheumatica.

Depending on jurisdiction, the invention can be worded as the use of rituximab and a glucocorticoid in the manufacture of a medicament for the treatment of polymyalgia.

Alternatively, depending on jurisdiction, the invention can be worded as a method for treating a patient with rituximab and a glucocorticoid, wherein the patient is suffering from polymyalgia rheumatica.

There are several well-known drawbacks to GC treatment including GC-related adverse events and contra-indications because of comorbidities. It is hence an object of the invention to reduce the treatment duration with GCs and/or to lower the GC dose. It is hereby understood that reducing the treatment duration should be interpreted as the use of rituximab to shorten the duration of the GC treatment and/or administration compared to a treatment with GC wherein rituximab is not administered. Similarly, it is understood that lowering the GC dose should be interpreted as the use of rituximab to lower the dose of the GC treatment compared to a treatment with GC wherein rituximab not is administered. Furthermore, it is understood that a dose has its normal meaning as used in the art. It refers to a certain amount of a medicine and may also include the time interval of the use of the medicine. Hence a dose can be expressed as an amount or as an amount per time interval, such as an amount per day or per week. It may even be expressed as an amount per abstract time interval, such as an amount per cycle and/or course or an amount per treatment. Also it is an object of the invention to achieve GC-free remission for PMR patients quicker. Hence, rituximab is used to achieve GC-free remission in PMR patients quicker compared to PMR patients who receive a GC treatment without rituximab.

Agents that allow for the reduction of the dose of a certain medicine without compromising the effectiveness can be denoted as sparing-agents. In this light, rituximab can be considered a GC sparing-agent as it allows for lowering the GC dose whilst the effectiveness of the rituximab/GC combination is comparable to or better than the GC at a higher dose. Hence, an aspect of the invention covers rituximab and a glucocorticoid for use in the treatment of polymyalgia rheumatica, wherein the rituximab is used to lower the glucocorticoid dose and/or as a glucocorticoid sparing-agent. It is understood that lowering the glucocorticoid dose refers to lowering the glucocorticoid dose when a combination of rituximab and GC is used compared to using the GC without the administration of rituximab.

It is also understood that according to the current invention, rituximab can act as a sparing-agent for GCs in the sense that it leads to significant shorter PMR disease duration meaning that the GC administration time is shortened resulting in a lower total dose of GC. Rituximab is hence used as GC sparing-agent wherein the PMR treatment is shortened compared to a treatment wherein rituximab is not administered.

It is common practice for PMR treatments where a GC is administrated to utilize a medication scheme where the dose of GC per administration is lowered whenever possible (for instance when the PMR symptoms are absent or are considered acceptable) and which limits the duration of the treatment as much as possible. In other words, the glucocorticoid administration scheme is chosen such that the dose and the duration of the treatment is minimized. It is understood that in the art, such an administration scheme where the dose is gradually lowered to reach zero is known as a tapering scheme.

Hence the glucocorticoid sparing may involve a glucocorticoid tapering scheme wherein the glucocorticoid is administered regularly, preferably daily, and preferably wherein the weekly average of the daily dose is lowered every 4 weeks, more preferably every 3 weeks and even more preferably every 2 weeks. It is hereby understood that weekly average of the daily dose refers to the sum of all administrations in a week divided by the number of days in a week (i.e. by 7).

In a further aspect of the disclosure, the glucocorticoid sparing involves a 10 - 52 week tapering scheme, more preferably a 12 - 42 week tapering scheme, even more preferably a 14 - 32 week tapering scheme, and most preferably a 15 - 25 week tapering scheme. It is hereby understood that the tapering scheme commences on the moment of the first GC administration. This is preferably on the same day as the rituximab administration and soon after the PMR diagnosis.

In an aspect, the glucocorticoid is at least one of cortisone, dexamethasone, hydrocortisone, methylprednisolone, prednisone, methylprednisone, prednisolone or methylprednisolone, triamcinolone, fludrocortisone, dexamethasone, or hydrocortisone. Preferably the glucocorticoid is prednisone, methylprednisone, prednisolone or methylprednisolone. More preferably the glucocorticoid is prednisone or prednisolone.

Rituximab is particularly useful for patients diagnosed with PMR and having a contra-indication for treatment with GC, for patients having a strong preference against treatment with GC and/or for patients having a bad prognosis with regard to glucocorticoid tapering. Rituximab is suitable for patients diagnosed with PMR and having a contra-indication for treatment with GC wherein the contra-indication includes an active current infection, a hepatitis B or tuberculosis infection, a state of severe immunodeficiency, severe heart failure (NYHA class IV), and/or a relative contra-indication such as hypertension, diabetes, cataract, and/or glaucoma.

A common complication during the treatment of PMR is the occurrence of flares. This applies particularly when a glucocorticoid tapering scheme is used, as the GC tapering scheme induces an increased risk of flares. In the context of the current disclosure, it is understood that flares relate to a sudden reoccurrence of symptoms and/or to the sudden increase in severity of the symptoms. When a flare is diagnosed, the treating physician often responds by increasing the glucocorticoid dose to suppress the re-occurrence and/or the increase in severity of the symptoms.

Rituximab may be used to reduce the occurrence of flares during the treatment of polymyalgia rheumatica. For instance, rituximab may be used to reduce the occurrence of flares during GC tapering and/or to manage the risk of prolonging the GC treatment as a result of flares.

It may thus be considered important to lower the glucocorticoid dose, preferably to reduce the incidence of GC related adverse events. Rituximab may be used to lower the glucocorticoid dose, preferably to reduce the incidence of GC related adverse events during the treatment of polymyalgia rheumatica. It is understood that lowering the glucocorticoid dose should be interpreted in the sense that the patient receives a lower dose of glucocorticoid than the patient would have received without the rituximab treatment. Preferably the lower dose of glucocorticoid is 80%, preferably 70%, more preferably 60% and most preferably 50%, of the corresponding dose without rituximab treatment. Alternatively formulated the lower dose is at most 25 mg GC/day, preferably at most 15 mg GC/day, more preferably 10 mg GC/day, even more preferably 7 mg GC/day and most preferably 5 mg GC/day.

It may be considered important to reduce the duration of the glucocorticoid treatment. Rituximab may be used to reduce the duration of the glucocorticoid treatment. It is understood that reducing the duration of the glucocorticoid treatment should be interpreted in the sense that the treatment with the combination of rituximab and the glucocorticoid is shorter than a corresponding treatment without the rituximab administration. It is understood that shorter is used to indicate a shorter time of treatment, such a reduction in treatment days. Preferably the treatment with reduced duration has 80%, preferably 70%, more preferably 60% and most preferably 50%, of the treatment days compared to a corresponding treatment without rituximab treatment.

Also it may be considered important to reduce disease activity and/or the disease burden without increasing the GC dose. Rituximab may be used reduce disease activity and/or the disease burden without increasing the GC dose. It is understood that reducing the disease activity should be interpreted in the sense that the symptoms of the disease are lower or further suppressed. In this respect lower symptoms or further suppressed symptoms can be interpreted as the patient having a perceived decrease in symptoms. Alternatively it can be interpreted as a lower score on the PMS-AS (see below). The phrase without increasing the GC dose should be interpreted in the sense that the patient does not receive a higher dose of glucocorticoid than the patient would have received without the administration of rituximab.

Also it may be considered important to improve the quality of life (QoL) of polymyalgia rheumatica patients. Rituximab may be used to improve the quality of life (QoL) of polymyalgia rheumatica patients. Hereby it is understood that QoL is used in its usual meaning as defined by the WHO. It is understood that improving the QoL is interpreted in the sense that the quality of life of patient receiving rituximab is improved compared to the QoL of patients that are not receiving rituximab.

PMR patients often suffer from morning stiffness. Rituximab may be used for the treatment of PMR patients wherein the morning stiffness that a PMR patient experiences is lowered. Hereby it is understood that lowering morning stiffness means that morning stiffness that a PMR patient experiences is lowered compared to the morning stiffness that a PMR patient not receiving rituximab experiences.

PMR patients often suffer from pain, such as muscle pain. Rituximab may be used for the treatment of PMR patients, wherein pain such as muscle pain is lowered. Hereby it is understood that lowering pain means that pain attributed to PMR is lowered compared to a PMR patient not receiving rituximab.

In an alternative or further aspect, the treatment further encompasses the administration of methotrexate (MTX) or leflunomide (LEF), wherein methotrexate is further preferred. MTX and LEF are considered rescue therapies for flaring PMR. In this respect, they can be used to support the PMR treatment and to further suppress flares. MTX is preferably administered in a therapeutically effective amount such as a dose of 5 - 50 mg/week or more preferably 15 - 25 mg/week. LEF is preferably administered in a therapeutically effective amount such as a dose of 30 - 300 mg/week, more preferably 500 - 200 mg/week or more preferably 70 - 140 mg/week.

To quantify the disease severity of polymyalgia rheumatica, an activity score has been developed in the art (see Leeb and Bird; A disease activity score for polymyalgia rheumatica; Ann Rheum Dis 2004, which is herewith incorporated entirely). This activity score - which may be referred to as the polymyalgia rheumatica activity score or the polymyalgia rheumatica disease activity score and which may be abbreviated as PMR-AS - provides an easily applicable and valid tool for monitoring PMR activity, and in combination with the PMR response criteria, provides an accurate description of response. The PMS-AS is a composite score of C-reactive protein level (CRP, mg/dl), the duration of morning stiffness (MST, minutes), the ability to raise the arms ((Elevation upper limb; EUL); 3 to 0: 3= no elevation possible; 2= elevation possible below shoulder girdle; 1= elevation possible up to shoulder girdle, 0= elevation possible above shoulder girdle), physician's global assessment (physician's visual analog scale (VASph); 0 to 10), and the patients' assessment of pain (patient's visual analog scale (VASp); 0 to 10). The total score can be calculated using the following expression: CRP (mg/dl)+VASp (0-10)+VASph (0-10)+(MST (min)^{∗}0.1)+EUL (3-0). It is understood that C-reactive protein is a protein which is known the art to increase in circulating concentrations reflecting an inflammation.

GC-free remission may be defined as polymyalgia rheumatic activity score of below a certain threshold value. Preferably GC-free remission is defined as a PMR-AS of lower of below 20, more preferably below 15, even more preferably below 12, still more preferably below 10, and most preferably below 7. Hence, rituximab may be employed to lower the polymyalgia rheumatica activity score. Preferably rituximab is used to treat a polymyalgia rheumatica patient to achieve a polymyalgia rheumatica activity score of below 20, more preferably below 15, even more preferably below 12, still more preferably below 10, and most preferably below 7. Rituximab, preferably in combination with a GC, may be used to achieve GC-free remission faster than a PMR treatment with GC where rituximab is not administered. Rituximab, preferably in combination with a GC, may be used in PMR treatment to lower the PMR-AS faster than a PMR treatment with GC where rituximab is not administered.

Rituximab is known as a medicine that shows effectiveness starting a few weeks after its initial dose and that is long-lasting. This means that rituximab can be administered on a monthly, bimonthly, half-yearly or even yearly base. It is hence understood that rituximab may be administered at most every month, preferably at most every 2 months, more preferably at most every 4 months, even more preferably at most every 6 months.

Alternatively, rituximab may only need to be administered at most 5 times during a full treatment, preferably at most 4 times, more preferably at most 3 times, still even more preferably at most 2 times, and most preferably only once during treatment. Hereby it is understood that at least one of the administrations is at the beginning of the rituximab treatment and that preferably the start of the treatment is defined as the time of administration of rituximab.

Hence, in an aspect, the rituximab is administered at beginning of the treatment, which is preferably within 4 weeks, preferably within 2 weeks, more preferably withing a week of the diagnosis of PMR. In an aspect, rituximab is administered as a single dose, preferably directly or early after diagnosis of polymyalgia rheumatica. In an aspect, rituximab is also administered after a flare has been diagnosed or when a recurrence of PMR is diagnosed. In an aspect, the rituximab is only administered when a flare or recurrence of PMR has been diagnosed. It is understood that the diagnosis of PMR and/or of a flare during PMR is preferably made by a rheumatologist or internal medicine specialist.

Rituximab is administered in a therapeutically effective amount. The dose of rituximab that is administered per course can be 50 - 3000 mg, preferably 100 - 2000 mg, more preferably 200 -15000 mg, even more preferably 300 - 1200 mg, still even more preferably 350 - 1000 mg and most preferably 500 -1000 mg. It is hereby understood that a course can be a single administration but may also consist of multiple administrations, such as 2, 3, or 4 administrations. For the current invention, a course is preferably 1 or 2 cycles. A course may also be referred to as a cycle.

The total cumulative dose of rituximab is preferably 200 - 5000 mg per treatment, wherein it is understood that the total dose equals the sum of all doses or administrations giving during a treatment and the treatment refers to the period between the first administration of a PMR treating medicament, which is preferably rituximab, to the moment the moment that it is decided to stop the administration of all PMR medicaments, for instance because the symptoms have disappeared. It is further preferred that the total dose of rituximab is 400 - 4000 mg per treatment, more preferably 600 - 3000 mg per treatment and even more preferably 800 - 2000 mg per treatment. It is preferred that a single dose of rituximab is 100 - 1500 mg, more preferably 200 - 1300 mg, even more preferably 400 - 1200 mg, still more preferably 500 - 1000 mg of rituximab.

During the treatment of PMR, rituximab is preferably administered by a rheumatologist or internal medicine specialist. Rituximab may be administered using any suitable method known in the art, wherein intravenous administration and/or subcutaneous administration may be preferred. It is hereby understood that an intravenous administration, such as an intravenous drip or intravenous injection, may be preferred as it is believed that this method of administration is more effective than other methods. Alternatively subcutaneous injection may be preferred as it is simple to perform and can even be self-administered. The glucocorticoid may also be administered using any suitable method known in the art, which includes oral administration, intravenous administration, intramuscular administration, intra-articular administration or topical administration. Preferably the GC is administered orally as this is simple, non-invasive and can be self-administered. Glucocorticoids are known to attribute to osteoporosis. Hence in an aspect, an osteoporosis prophylaxis agent is administered along with the glucocorticoid.

If necessary, the patients that are planned to receive the novel rituximab treatment may receive a premedication comprising of a single dose of 25-100 mg methylprednisolone (which may be administered intra-venously), 500-1500 mg acetaminophen (which may be administered orally), and antihistamines (such as 5-15 mg orally administered cetirizine, or 0.5-1.5 mg clemastine which is administered intra-venously). For subcutaneous administration, premedication may not be necessary.

It will be clear to the skilled person that various (preferred) objects, aspects and embodiments described herein can be combined, unless they are mutually exclusive.

It must be noted that as used herein and in the appended claims, the singular forms "a", "an", and "the" include plural references unless the context clearly dictates otherwise. Furthermore, the terms "a" (or "an"), "one or more" and "at least one" can be used interchangeably herein. The terms "comprising", "including", "having" and "constructed from" can also be used interchangeably.

The invention is illustrated by the following examples, without being limited thereto or thereby.

### EXAMPLES

### Example 1

A 21-week, double-blind placebo controlled trial was conducted wherein the efficacy of rituximab for the treatment of PMR was examined. The study was conducted at the Department of Rheumatology at the Sint Maartenskliniek, The Netherlands. Medical ethical approval was obtained from the Medical ethical committee of the region Arnhem-Nijmegen and the local research committee. This study followed the CONSORT guidelines for conducting and reporting Randomized Controlled Trials (RCTs). The CONSORT guidelines are generally accepted evidence-based guidelines for RCTs which are publicly available at http://www.consort-statement.org/. All eligible or interested participants received written and oral information on the study and the risks involved by participating in the study. All participants gave written informed consent before study inclusion. The trial was monitored according to good clinical practice by an independent monitor before, during and after the study period. Additionally, a Data Safety Monitoring Board was installed (DSMB) which held three-monthly meetings in order to safeguard the feasibility of the study and safety of the study participants.

Eligible patients included newly, recently diagnosed and relapsing PMR patients according to the 2012 EULAR/ ACR PMR classification criteria. The inclusion criteria for recently diagnosed PMR patients included a recent polymyalgia rheumatica diagnosis (within the past 3 months) according to the 2012 EULAR/ ACR polymyalgia rheumatica classification. The newly and recently diagnosed PMR patients were further:
- glucocorticoid naive (newly diagnosed), or,
- receiving glucocorticoid for less than 6 consecutive weeks before study inclusion with a maximum daily dose of 20mg, or,
- on a short treatment of 30mg for 7 days and / or methylprednisolone intramuscular not more than 120 mg in the past 3 months.

Relapsing PMR patients were eligible if the relapse occurred at a daily GC dose of ≥7.5 mg. Main exclusion criteria for all patients were a daily dose of oral GC exceeding 30 mg, exposure to other immunosuppressant treatments in the past 4 months before study inclusion, other concomitant inflammatory rheumatic diseases or other diseases that may have hampered adequate assessment of PMR. Prior to receiving the study intervention, patients were actively screened for hepatitis B, tuberculosis or concurrent GCA.

Patients were randomly allocated to receive RTX or placebo (PCB). The randomization scheme was generated by an independent pharmacist using a computerized randomization procedure with varying block size (two blocks of 20 subjects, and one block of 10 subjects) to ensure allocation concealment of the intervention.

Patients in the intervention arm received 1 x 1000 mg RTX and patients in the placebo arm 1x0 mg RTX (sodium chloride 0.9%), within three weeks after enrolment of the study. Two vials of each 50 mL containing concentrated 500mg of RTX (Rixathon; Sandoz bv) were used for solution for infusion. This was intravenously administered after dilution in 500 mL sodium chloride 0.9%. PCB consisted of only 500 mL sodium chloride 0.9%. It was visually impossible to distinguish RTX from PCB. The standard premedication comparable to the treatment protocol in RA was given in both groups to ensure blinding of the intervention. This consisted of a single dose of intravenous 50 mg methylprednisolone, acetaminophen 1000 mg and antihistamines (cetirizine 10 mg) prior to the infusion.

All patients received the same prednisolone treatment and in total, prednisolone was given for a minimum of 17 weeks from the day of intervention (See Table 1).

**Table 1: Prednisolone tapering schedule, dose in mg, initiated at date of rituximab /placebo**

| | Day 1 | Day 2 | Day 3 | Day 4 | Day 5 | Day 6 | Day 7 |
|---|---|---|---|---|---|---|---|
| Week 1 | 50* | 15 | 15 | 15 | 15 | 15 | 15 |
| Week 2 | 15 | 15 | 15 | 15 | 15 | 15 | 15 |
| Week 3 | 15 | 15 | 15 | 15 | 15 | 15 | 15 |
| Week 4 | 15 | 15 | 15 | 15 | 15 | 15 | 15 |
| Week 5 | 10 | 15 | 15 | 15 | 15 | 15 | 15 |
| Week 6 | 10 | 15 | 10 | 15 | 15 | 15 | 15 |
| Week 7 | 10 | 15 | 10 | 15 | 10 | 15 | 15 |
| Week 8 | 10 | 15 | 10 | 15 | 10 | 15 | 10 |
| Week 9 | 10 | 10 | 10 | 15 | 10 | 15 | 10 |
| Week 10 | 10 | 10 | 10 | 10 | 10 | 15 | 10 |
| Week 11 | 10 | 10 | 10 | 10 | 10 | 10 | 10 |
| Week 12 | 7,5 | 7,5 | 7,5 | 7,5 | 7,5 | 7,5 | 7,5 |
| Week 13 | 7,5 | 7,5 | 7,5 | 7,5 | 7,5 | 7,5 | 7,5 |
| Week 14 | 5 | 5 | 5 | 5 | 5 | 5 | 5 |
| Week 15 | 5 | 5 | 5 | 5 | 5 | 5 | 5 |
| Week 16 | 2,5 | 2,5 | 2,5 | 2,5 | 2,5 | 2,5 | 2,5 |
| Week 17 | 2,5 | 2,5 | 2,5 | 2,5 | 2,5 | 2,5 | 2,5 |
| Week 18 | 0 | 0 | 0 | 0 | 0 | 0 | 0 |
| Week 19 | 0 | 0 | 0 | 0 | 0 | 0 | 0 |
| Week 20 | 0 | 0 | 0 | 0 | 0 | 0 | 0 |
| Week 21 | 0 | 0 | 0 | 0 | 0 | 0 | 0 |

| | | | | | | | |
|---|---|---|---|---|---|---|---|
| * All patients were administered intravenous methylprednisolone 50 mg prior to infusion of rituximab / placebo to prevent allergic reactions of the intervention, following local protocol of rituximab infusions. | | | | | | | |

As it was expected that RTX would show efficacy after 8-11 weeks, it was decided to start with a regular GC tapering scheme, and accelerate (compared to the regular scheme) the prednisolone tapering from week 11 onwards. In case that a patient experienced a flare after initial response (secondary non-responder), prednisolone was increased up to the last effective dose for two weeks and tapered in accelerated manner after remission. In case that a second flare occurred, the prednisolone was again increased to the last effective dose and after two weeks subsequently tapered according to the slower usual care tapering scheme following the local protocol. If a patient experienced a recurrence of disease after cessation of prednisolone, it was reinitiated and subsequently tapered according to above mentioned manner. At the beginning of the study, all patients received adequate osteoporosis prophylaxis.

Visits and assessment of study outcomes took place at baseline, week 2, 4, 11, 17 and 21. Extra visits were planned if patients experienced an increase in possible PMR symptoms. Aside from baseline patient, disease characteristics, measurements, laboratory values and imaging, we also collected data on disease activity and asked patients to fill in functional and health related questionnaires, described in more detail below.

The primary outcome was proportion of GC-free remission at week 21 after RTX infusion, in both groups. GC-free remission is defined as polymyalgia rheumatic activity score (PMR-AS) < 10, indicating low disease activity as proposed by earlier studies. The PMR-AS is a composite score of the following items: CRP level (mg/dl), the duration of morning stiffness (MST, minutes), the ability to raise the arms ((Elevation upper limb; EUL); 3 to 0: 3= no elevation possible; 2= elevation possible below shoulder girdle; 1= elevation possible up to shoulder girdle, 0= elevation possible above shoulder girdle), physician's global assessment (physician's visual analog scale (VASph); 0 to 10), and the patients' assessment of pain (patient's visual analog scale (VASp); 0 to 10). The total score was calculated with the following equation: CRP (mg/dl)+VASp (0-10)+VASph (0-10)+(MST (min)^{∗}0.1)+EUL (3-0). We defined a flare as judged by the treating physician, if symptoms and / or raised ESR and CRP recurred that were most likely attributable to PMR.

Pre-planned secondary outcomes at week 21 were between group differences in (change in) ESR/CRP, PMR-AS, biomarkers, cumulative GC dose, and the proportion of patients achieving remission on a low dose of GC (5 mg or less). Additionally, functional status and quality of life were assessed by using the HAQ disability index (HAQ-DI), the HAQ's patient global and pain visual analog scales (VAS), patient stiffness VAS, patient's global fatigue VAS, EQ5D-5L and the SF-12. Post hoc analyses included the total and per treatment group proportion of GC-free remission in patients who were either recently/newly diagnosed PMR patients or had a PMR flare at moment of study inclusion. Secondly, the proportion of GC-free remission as judged by the treating research physician was assessed. Lastly, the proportion of low-dose GC use at the end of the study in patients who were either recently/newly diagnosed PMR patients or had a PMR flare at moment of study inclusion was assessed.

In this exploratory study we calculated a sample size of 25 per treatment arm so 50 patients in total, including holding into account a drop-out of 5%. With a comparison of the proportion of patients in GC-free remission with a Fischer's exact test and one sided alpha level of 0.05, this sample size gives enough power (at least 86%) to assess a relevant difference in effect of 40% points in the advantage of RTX, independent of the response of prednisolone only group. Table 2 below shows the power of one sided Fisher exact test with alpha 0.05 with a sample size of 25 patients per group with different effect sizes and response percentages on prednisolone alone for different effect sizes and response percentages.

**Table 2: Power of one sided Fisher exact test with alpha 0.05 with a sample size of 25 patients per group with different effect sizes and response percentages on prednisolone alone**

| | **Response in control group** | | | |
|---|---|---|---|---|
| **Treatment effect (risk difference for response in favour of RTX)** | **20%** | **30%** | **40%** | **50%** |
| 20% | 36% | 34% | 34% | 34% |
| 30% | 63% | 62% | 63% | 63% |
| 40% | 87% | 86% | 87% | 91% |

At 21 weeks, significantly more patients in the RTX group achieved GC-free remission (PMR-AS<10) compared to the PCB group (48% versus 21%, 95%-Cl 0.4% to 54%, 1-sided Fischer's exact p=0.049). Also the mean PMR-AS was lower for the RTX group than for the PCB group as shown in Table 3.

**Table 3: mean PMR-AS as function of week for the RTX group compared to the PBO group.**

| **Week** | Mean PMR-AS | |
|---|---|---|
| | **RTX** | **PBO** |
| 0 | 21,4 | 17,9 |
| 2 | 4,2 | 6,5 |
| 4 | 2,9 | 6,0 |
| 11 | 2,0 | 7,9 |
| 17 | 6,3 | 10,9 |
| 21 | 7,7 | 14,0 |

Additionally, low-dose GC at 21 weeks was assessed. At 21 weeks, significantly more patients in the RTX group achieved low-dose GC compared to the PCB group (91% versus 54%, 95%-Cl 11% to 63%, 1-sided Fischer's exact p=0.005). In the post-hoc analysis, recently/newly diagnosed PMR patients treated with rituximab vs placebo were more likely to achieve GC free remission (58% versus 21%, 95%-Cl 5% to 68%, 1-sided Fischer's exact p=0.02), compared to relapse patients treated with rituximab vs placebo (0 versus 20%, 95%-Cl -64 to 24%, 1-sided Fischer's exact p=0.56). Also, regarding the secondary outcome low dose glucocorticoid, the effect of rituximab vs placebo was greater in recently diagnosed polymyalgia rheumatica patients: 95% versus 47%, 95%-Cl of difference 18 to 77%, 1-sided Fischers' exact p=0.002 than relapse patients vs placebo (75% versus 80%, 95%-Cl of difference -63 to 53%, 1-sided Fischers' exact p=0.72).

### Example 2

A 26-week, double-blind placebo controlled trial is conducted wherein the efficacy of rituximab for the treatment of PMR is examined. Eligible patients include newly, recently diagnosed and relapsing PMR patients according to the 2012 EULAR/ ACR PMR classification criteria. Patients are randomly allocated to receive rituximab (RTX), standard prednisolone treatment (PRDL) or a placebo (PCB).

Patients in the RTX arm receive 2 x 500 mg RTX in week 0 and 2 x 500 mg RTX in week 12 along with a placebo mimicking a PRDL treatment (1 pill per day). Patients in the PRDL arm receive 2x0 mg RTX (Sodiumchloride 0.9%) at week 0 and week 12 along with PRDL according to the tapering scheme of Table 1. This is administered as 1 pill per day. Patients in the PCB arm receive 2x0 mg RTX (Sodiumchloride 0.9%) at week 0 and week 12 along with a placebo mimicking a PRDL treatment (1 pill per day). The standard premedication comparable to the treatment protocol in RA is given in both groups to ensure blinding of the intervention. This consists of a single dose of intravenous 50 mg methylprednisolone, acetaminophen 1000 mg and antihistamines (cetirizine 10 mg) prior to the infusion.

## Claims

1. Rituximab for use in the treatment of polymyalgia rheumatica.

2. Rituximab for use according to claim 1, wherein the treatment further comprises the administration of a glucocorticoid, preferably wherein the rituximab is used to lower the glucocorticoid dose and/or as a glucocorticoid sparing agent.

3. Rituximab for use according to claim 2, wherein the rituximab is used to reduce the duration of the treatment with the glucocorticoid.

4. Rituximab for use according to claim 2 or 3, wherein the glucocorticoid sparing comprises a 10 - 52 week, preferably 12 - 42 week, more preferably 14 - 32 week, even more preferably 15 - 25 week glucocorticoid tapering scheme.

5. Rituximab for use according to any of the previous claims, wherein the patient diagnosed with polymyalgia rheumatica has a contra-indication for treatment with glucocorticoids, a strong patient preference against treatment with glucocorticoids and/or a bad prognosis with regard to glucocorticoid tapering.

6. Rituximab for use according to any of the previous claims, wherein the rituximab is used to reduce the incidence of flares.

7. Rituximab for use according to any of the previous claims, wherein the rituximab is used to reduce the disease duration of polymyalgia rheumatica.

8. Rituximab for use according to any of the previous claims, wherein the rituximab is used to reduce the recurrence of polymyalgia rheumatica.

9. Rituximab for use according to any of the previous claims, wherein the rituximab is used to lower the polymyalgia rheumatica activity score or the polymyalgia rheumatica disease activity score.

10. Rituximab for use according to any of the previous claims, wherein the rituximab is administered in less than 5 doses, preferably within 4 weeks after diagnosis of polymyalgia rheumatica.

11. Rituximab for use according to any of the previous claims, wherein the total cumulative dose of rituximab is 200 - 5000 mg.

12. Rituximab for use according to any of the previous claims, wherein the dose of rituximab is 50 - 3000 mg per course.

13. Rituximab for use according to any of the previous claims, wherein the rituximab is administered intravenously or by subcutaneous injection.

14. Rituximab for use according to any of claims 2 - 14, wherein the glucocorticoid is administered orally.

15. Rituximab for use according to any of claims 2 - 15, wherein the glucocorticoid is cortisone, dexamethasone, hydrocortisone, methylprednisolone , prednisone, methylprednisone, prednisolone or methylprednisolone, triamcinolone, fludrocortisone, dexamethasone, or hydrocortisone, preferably wherein the glucocorticoid is prednisone, methylprednisone, prednisolone or methylprednisolone.
